# EUROPEAN PATENT APPLICATION

(11) **EP 4 183 389 A1**
(43) Date of publication of application: **24.05.2023**
(21) Application number: 21209153.2
(22) Date of filing: 18.11.2021
(51) Int. Cl.: A61K 9/20, A61K 31/4035, A61P 17/06, C30B 1/02

(54) **PHARMACEUTICAL COMPOSITION COMPRISING APREMILAST**

(71) Applicant: KRKA, d.d., Novo mesto, 8000 Novo mesto (SI)
(72) Inventor: LORBEK, Gregor, 8501 Novo Mesto (SI); PAVLIN, Darja, 8501 Novo Mesto (SI); HOTKO, Anka, 8501 Novo Mesto (SI); KORASA, Klemen, 8501 Novo Mesto (SI); BENKIC, Primoz, 8501 Novo Mesto (SI); BEZENSEK, Jure, 8501 Novo Mesto (SI); GOGNJAVEC, Tjasa, 8501 Novo Mesto (SI); PUSAVEC KIRAR, Eva, 8501 Novo Mesto (SI)
(74) Representative: Hoefer & Partner Patentanwälte mbB

(57) **Abstract**

The present invention relates to a pharmaceutical composition comprising apremilast, as well as to a process for the preparation thereof. Furthermore, the present invention pertains to a method for crystallizing apremilast, and to apremilast obtainable by this method. Furthermore, apremilast and pharmaceutical compositions for use as a medicament are disclosed.

## Description

### Field of the invention

The invention relates to a pharmaceutical composition comprising apremilast, as well as to a process for preparing apremilast and to apremilast obtainable by this process.

### Background of the invention

Apremilast is an active ingredient having advantageous pharmacological properties. Apremilast is also referred to herein as (+)-2-[1-(3-ethoxy-4-methoxy-phenyl)-2-methylsulfonyl-ethyl]-4-acetylaminoisoindoline-1,3-dione. Apremilast has the chemical formula (A), which is shown below:

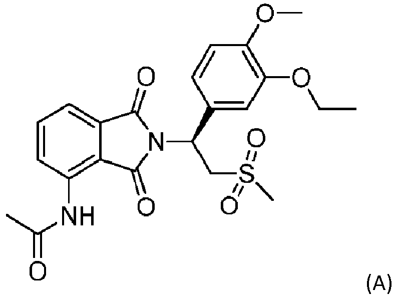

Apremilast is a compound with anti-inflammatory activity, and is used for the treatment or prevention of a variety of diseases and conditions, such as inflammatory conditions, psoriasis and Behçet's disease.

Apremilast has been described as blocking the degradation of cyclic adenosine monophosphate (cAMP) via inhibition of the phosphodiesterase type IV (PDE4) enzyme, resulting in an increase in cAMP in PDE4-expressing cells including monocytes, T cells, and neutrophils. Preparation methods being e.g. indicated in U.S. Patent No. 6,962,940.

Commercially available compositions comprising apremilast are marketed under the name Otezla^{®}.

WO 2013/101810 A1 describes a pharmaceutical composition comprising a compound for formula (A), a filler, a disintegrant and a lubricant, wherein the filler is lactose. The specific compositions given in Table 8 of WO 2013/101810 A1 are either identical with or largely similar to commercially available compositions marketed under the name Otezla^{®}.

Further documents describing oral pharmaceutical compositions comprising apremilast are US 6,962,940 B2, US 2007/0155791 A1, WO 2014/072259 A1. WO 2014/204825 A1, WO 2017/076987 A1, WO 2019/073331 A2, WO 2019/073477 A1, WO 2019/116386 A1, WO 2020/242414 A1.

Although WO 2013/101810 A1, US 6,962,940 B2 and US 2007/0155791 A1 already disclose several pharmaceutical compositions comprising apremilast, there still exists a need in the art to prepare pharmaceutical compositions which overcome the drawbacks of the known formulations. In particular, there is still a need in the art for pharmaceutical compositions comprising apremilast which may be advantageously prepared, especially pharmaceutical compositions which are obtainable from mixtures having an advantageous flowability, an advantageous compressibility, and an advantageous low sticking tendency, especially during preparation procedures involving compression procedures. Furthermore, there is still a need in the art to provide pharmaceutical compositions, which avoid or reduce the amount of excipient(s) which might be disliked or might be not well tolerated by some groups of persons in need of treatment or prevention with apremilast, in particular to avoid or reduce lactose. Moreover, there is a need in the art to prepare a pharmaceutical composition comprising apremilast and excipients, which is particularly adapted to a preparation process comprising direct tableting.

Additionally or alternatively, the present inventors also aimed at providing apremilast having improved properties, especially improved properties for the production of pharmaceutical compositions, in particular improved properties for preparation processes involving subjecting a mixture to compression. Advantageous compression properties and a low sticking tendency to devices used during production processes is in particular of importance when preparing compressed mixtures, e.g. tablets (in particular tablets prepared by direct compression) or granules. Furthermore, there is a need in the art to obtain apremilast having an advantageous morphology (especially an absence or reduced amount of particles in needle form), and/or desired physicochemical properties (especially advantageous flow properties, and/or an advantageous resistance of the apremilast particles to mechanical stress (e.g. for ensuring that the particles do not crumble during the preparation process), and/or an advantageous dissolution rate (especially a dissolution rate which remains advantageous or acceptable after exposure to stress conditions (increased temperature (e.g. 50°C) or increased relative humidity (e.g. 75%)). In particular, there is a need in the art for an apremilast preparation method which allows to obtain apremilast having a particle size overcoming the need for apremilast grinding procedures during pharmaceutical composition preparation. Furthermore, the apremilast preparation method should allow obtaining apremilast which merely contains apremilast agglomerates and/or apremilast particles having needle form in low amount or is free of apremilast agglomerates and/or apremilast particles having needle form.

Unexpectedly and surprisingly, pharmaceutical composition comprising apremilast solving the above-indicated problems were found by the present inventors.

### Brief Summary of the invention

According to a first aspect of the present invention, a pharmaceutical composition is provided, said pharmaceutical composition being or comprising a mixture, said mixture comprising:
3 - 20 wt.%, preferably 5 - 15 wt.%, more preferably 7.5 - 12.5 wt.% of apremilast, based on the total weight of said mixture,
40 - 90 wt.%, preferably 50 - 80 wt.%, more preferably 55 - 75 wt.% of mannitol, based on the total weight of said mixture,
0.5 - 3 wt.%, preferably 1 - 2 wt.%, more preferably 1.2 - 1.8 wt.% of magnesium stearate, based on the total weight of said mixture, and
0.5 - 6 wt.%, preferably 1 - 5 wt.%, more preferably 2 - 4 wt.% of a disintegrant, based on the total weight of said mixture.

Apremilast is also referred to herein as (+)-2-[1-(3-ethoxy-4-methoxy-phenyl)-2-methylsulfonylethyl]-4- acetylaminoisoindoline-1,3-dione. Apremilast has the chemical formula (A), which is shown below:

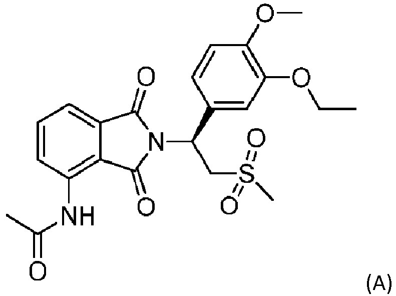

According to a further aspect of the present invention, a method for crystallizing apremilast is provided, said method comprising the following steps:
a) preparing a solution comprising apremilast and a solvent, said solvent being or comprising acetone,
b) optionally adding seeding material to the solution comprising apremilast and a solvent to obtain a seeding material containing mixture,
c) adding an antisolvent and optionally adding seeding material to the solution comprising apremilast prepared in step a) or to the seeding crystal containing mixture optionally prepared in step b) during an antisolvent addition time period of from 1 minute to 20 hours, preferably of from 10 minutes to 1 hour, to obtain a mixture comprising apremilast, solvent, and antisolvent,
   said antisolvent being or comprising ethanol,
d) optionally increasing the temperature of the mixture comprising apremilast, solvent, and antisolvent obtained in step c) to a temperature in the temperature range up to 65°C,
e) subjecting the mixture comprising apremilast, solvent, and antisolvent to agitation at a temperature in a first crystallization temperature range of from 35 °C to 55 °C, preferably from 40°C to 50°C, during a first crystallization period of from 5 hours to 25 hours, preferably of from 10 hours to 20 hours, to obtain a suspension comprising apremilast,
f) decreasing the temperature of the suspension comprising apremilast to a temperature in a second crystallization temperature range of from -20°C to 20°C, preferably of from -10°C to 10°C,
g) separating crystalline apremilast from the suspension comprising apremilast,
h) optionally drying the separated crystalline apremilast.

According to a still further aspect of the present invention, apremilast obtainable by the method for crystallizing apremilast of the present invention is provided.

### Figures

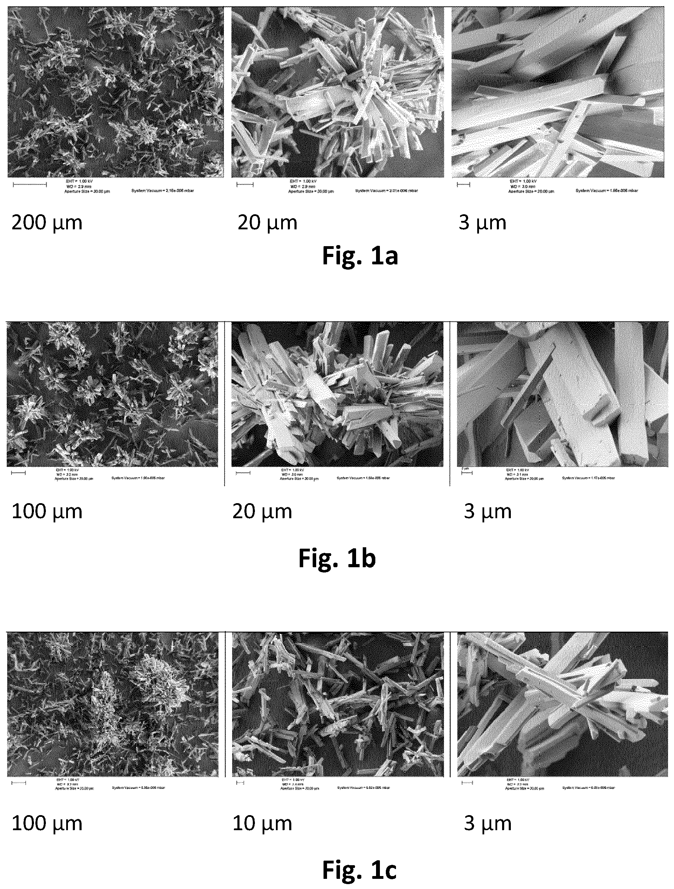

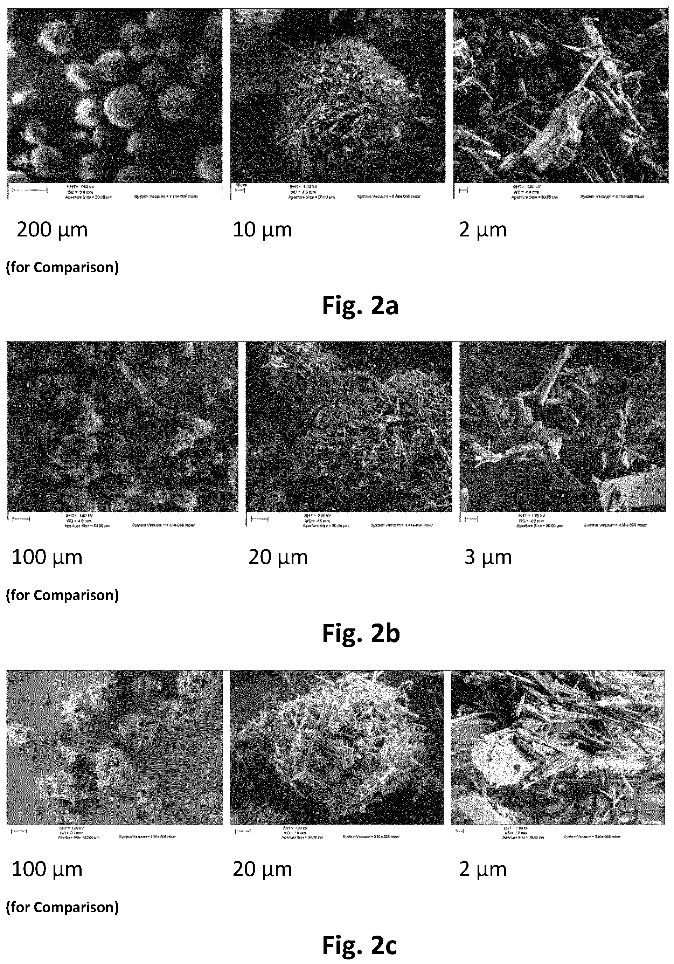

Figures 1a to 1d show SEM images of apremilast prepared according to the crystallization method of the invention. Figures 2a to 2c show SEM images of apremilast not prepared according to the crystallization method of the invention.

For each of Figures 1 a to 1c, and 2a to 2c the SEM image at the left side is at 200 x magnification, the SEM image in the middle is at 1000 x magnification, and the SEM image at the right side is at 5000 x magnification. In particular:
Figure 1a shows apremilast obtained according to Crystallization Example 1,
Figure 1b shows apremilast obtained according to Crystallization Example 2,
Figure 1c shows apremilast obtained according to Crystallization Example 3.
   For the SEM image on the left side of Fig. 1b, exemplarily the following determination parameters are indicated: EHT (electron high tension) = 1.00 kV, WD (working distance) = 3.2 mm, Aperture Size = 30.00 µm, System Vacuum = 2.05 e-005 mbar.
Figure 2a shows apremilast obtained according to Comparative Crystallization Example 1,
Figure 2b shows apremilast obtained according to Comparative Crystallization Example 2,
Figure 2c shows apremilast obtained according to Comparative Crystallization Example 3.
Figure 3 shows dissolution profiles comparing tablets having compositions according to the present invention with Otezla^{®} tablets.

### Detailed description of the invention

Unexpectedly, the present inventors found highly advantageous pharmaceutical compositions which provide several advantages. The pharmaceutical compositions of the present invention facilitate the preparation procedures, and thereby increase both cost and time efficiency of the preparation process. The pharmaceutical compositions are obtainable from mixtures having an advantageous flowability, an advantageous compressibility, and an advantageous low sticking tendency, especially a low sticking tendency to devices during preparation processes involving compression procedures.

Furthermore, the pharmaceutical compositions allow avoiding to use or to reduce the amount of lactose, especially lactose monohydrate. Pharmaceutical compositions, which are free of lactose or have a reduced content of lactose, are highly beneficial for persons avoiding the ingestion of lactose for various reasons (e.g. low lactose tolerance). Surprisingly, the present inventors found that pharmaceutical compositions comprising mannitol, in particular both mannitol and disintegrant, allow to avoid using lactose or to reduce the amount of lactose in pharmaceutical compositions comprising apremilast. Reducing the amount of lactose in compositions comprising apremilast is highly difficult, as lactose (in particular lactose monohydrate) is an excipient providing numerous advantageous properties, e.g. advantageous compressibility properties.

Moreover, pharmaceutical compositions of the present invention have appropriate dissolution properties, and a desired storage stability, as well as have an advantageous resistance against physical stress. Pharmaceutical compositions of the present invention prepared by processes using compression step(s) may be advantageously prepared applying low or moderate pressure during compression step(s).

According to a first aspect of the present invention, a pharmaceutical composition is provided, which pharmaceutical composition is or comprises a mixture (preferably said mixture being a compressed mixture), said mixture comprising:
3 - 20 wt.%, preferably 5 - 15 wt.%, more preferably 7.5 - 12.5 wt.% of apremilast, based on the total weight of said mixture, advantageously the apremilast being crystalline apremilast,
40 - 90 wt.%, preferably 50 - 80 wt.%, more preferably 55 - 75 wt.% of mannitol, based on the total weight of said mixture,
0.5 - 3 wt.%, preferably 1 - 2 wt.%, more preferably 1.2 - 1.8 wt.% of magnesium stearate, based on the total weight of said mixture, and
0.5 - 6 wt.%, preferably 1 - 5 wt.%, more preferably 2 - 4 wt.% of a disintegrant, based on the total weight of said mixture, advantageously the disintegrant being or comprising croscarmellose sodium,
and optionally 0.1 - 45 wt.%, preferably 10 - 35 wt.%, more preferably 15 - 30 wt.% of a filler, based on the total weight of said mixture, said filler being a filler other than a filler comprising or consisting of mannitol, advantageously the filler being or comprising microcrystalline cellulose.

The pharmaceutical composition of the present invention can be in particular a solid pharmaceutical composition. The mixture comprising apremilast, mannitol, magnesium stearate, and disintegrant in specific weight amounts (e.g. as defined in the claims), which is contained in the pharmaceutical composition of the present invention, can be hereinafter also referred to as "mixture comprising apremilast, mannitol, magnesium stearate, and disintegrant" or as "mixture comprising apremilast, and mannitol".

Advantageously, the pharmaceutical composition can be or can comprise a mixture comprising 3 - 20 wt.%, preferably 5 - 15 wt.%, more preferably 7.5 - 12.5 wt.% of apremilast,
50 - 80 wt.%, more preferably 55 - 75 wt.% of mannitol,
0.5 - 3 wt.%, preferably 1 - 2 wt.%, more preferably 1.2 - 1.8 wt.% of magnesium stearate, and
0.5 - 6 wt.%, preferably 1 - 5 wt.%, more preferably 2 - 4 wt.% of a disintegrant, each based on the total weight of said mixture, and
optionally 0.1 - 45 wt.%, preferably 10 - 35 wt.%, more preferably 15 - 30 wt.% of a filler, based on the total weight of said mixture, said filler being a filler other than a filler comprising or consisting of mannitol.

Especially, the pharmaceutical composition can be or can comprise a mixture comprising 3 - 20 wt.%, preferably 5 - 15 wt.%, more preferably 7.5 - 12.5 wt.% of apremilast,
55 - 75 wt.% of mannitol,
0.5 - 3 wt.%, preferably 1 - 2 wt.%, more preferably 1.2 - 1.8 wt.% of magnesium stearate, and
0.5 - 6 wt.%, preferably 1 - 5 wt.%, more preferably 2 - 4 wt.% of a disintegrant, each based on the total weight of said mixture.

In particular, the pharmaceutical composition can be or can comprise a mixture comprising 5 - 15 wt.%, more preferably 7.5 - 12.5 wt.% of apremilast,
50 - 80 wt.%, more preferably 55 - 75 wt.% of mannitol,
1 - 2 wt.%, more preferably 1.2 - 1.8 wt.% of magnesium stearate, and
1 - 5 wt.%, more preferably 2 - 4 wt.% of a disintegrant, each based on the total weight of said mixture.

Especially, the pharmaceutical composition can be or can comprise a mixture comprising 7.5 - 12.5 wt.% of apremilast,
55 - 75 wt.% of mannitol,
1.2 - 1.8 wt.% of magnesium stearate, and
2 - 4 wt.% of a disintegrant, each based on the total weight of said mixture.

Apremilast is a commercially available active ingredient, marketed as active ingredient in compositions e.g. under the name Otezla^{®} (Amgen). The preparation of apremilast being for example described in WO 03/080049 A1. The term apremilast as used herein can encompass both apremilast and solvate(s) thereof. As used herein, and unless otherwise specified, the term "solvate of apremilast" means apremilast that further includes a stoichiometric or non-stoichiometric amount of solvent(s) bound, e.g. by non-covalent intermolecular forces; preferably, the solvent(s) bound being solvent(s) which are pharmaceutically acceptable for oral ingestion. Where the solvent is water, the solvate is a hydrate.

For preparing the pharmaceutical composition of the invention, apremilast can be used in amorphous form or crystalline form or as an apremilast comprising apremilast in amorphous form and in crystalline form; preferably crystalline apremilast can be used. In particular, the mixture comprising apremilast, mannitol, magnesium stearate, and disintegrant, can be a mixture wherein the apremilast is or comprises apremilast in crystalline form or amorphous form or in form of a mixture comprising apremilast in amorphous form and crystalline form. Preferably, the mixture comprising apremilast, mannitol, magnesium stearate, and disintegrant, can be a mixture wherein the apremilast is or comprises apremilast in crystalline form, more preferably wherein the apremilast is apremilast in crystalline form.

Advantageously, the pharmaceutical composition of the invention comprises apremilast, which is apremilast prepared according to the method for crystallizing apremilast of the present invention (disclosed herein below).

The manufacturing process of the active substance can be advantageously designed to manufacture (+)-2-[1-(3-ethoxy-4-methoxy-phenyl)-2-methylsulfonyl-ethyl]-4- acetylaminoisoindoline-1,3-dione, whose enantiomeric purity can be routinely controlled by chiral HPLC. The process for preparing a pharmaceutical composition of the present invention can provide that no inter-conversion between apremilast and its enantiomer during the preparation process occurs. Furthermore, the crystallization method of the present invention can avoid an inter-conversion of (+)-2-[1-(3-ethoxy-4-methoxy-phenyl)-2-methylsulfonyl-ethyl]-4- acetylaminoisoindoline-1,3-dione into the (-)-enantiomer.

Advantageously, the pharmaceutical composition is or comprises a mixture which can further comprise 0.1 - 45 wt.%, preferably 10 - 35 wt.%, more preferably 15 - 30 wt.% of a filler, based on the total weight of the mixture, said filler being a filler other than a filler comprising or consisting of mannitol. Said filler can be in particular selected from the group consisting of microcrystalline cellulose, silicified microcrystalline cellulose, dextran, starches (such as corn starch), pregelatinized starch, xylitol, lactitol, sucrose, fructose, coprocessed fructose and starch, isomalt, maltodexrin, maltitol, alkali or earth alkali salts of phosphoric acid (such as calcium hydrogenphosphate), and combinations thereof. Preferably said filler is microcrystalline cellulose or a mixture comprising microcrystalline cellulose, and at least one further filler, said further filler being a filler other than a filler comprising or consisting of mannitol.

In particular, the pharmaceutical composition can be or can comprise a compressed mixture, in particular can be or can comprise a tablet. Optionally a coating, especially a film coating can be applied on the mixture, especially compressed mixture, in particular tablet. In a preferred embodiment, the pharmaceutical composition is a coated tablet which comprises a compressed mixture in the form of a tablet, and a coating coated onto the tablet. A compressed mixture can be a mixture which has been subjected to a compression procedure, such as a tablet, a (compressed) tablet layer in a layered tablet comprising 2 or 3 or more layers, a (compressed) core (e.g. tablet core), granule(s) obtained by a preparation process involving a compression process, e.g. dry granulate, especially granule(s) prepared by a process comprising roller compaction (also referred to herein as roller compacted granule(s)).

The pharmaceutical compositions of the present invention are or comprise a mixture comprising mannitol. Mannitol is a naturally sugar alcohol, and is described in European Pharmacopoeia, e.g. in European Pharmacopoeia 6.0, Volume 2. Further information on Mannitol can be found in the "Handbook of Pharmaceutical Excipients", 6th edition, ed. by R.C. Rowe et al., 2010 (reprinted), p. 424 - 428. The terms "mannitol" and "D-mannitol" can be used interchangeably herein. D-mannitol has the structural formula shown below:

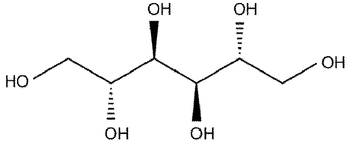

In particular, the pharmaceutical composition can be or can comprise a mixture, wherein the mannitol is or comprises spray-dried mannitol. Mannitol is commercially available e.g. under the tradename Parteck^{®} M100 or Parteck^{®} M200 (EMD Millipore Corporation, Billerica, USA).

Mannitol can be used in any form. Mannitol is preferably mannitol in powder form, especially spray-dried mannitol in powder form. For preparing pharmaceutical compositions of the present invention, especially for the preparation of the mixture (especially compressed mixture) comprising apremilast, mannitol, magnesium stearate and disintegrant contained therein, e.g. crystalline mannitol, granulated mannitol, spray-dried mannitol, and combinations thereof, preferably spray-dried mannitol, can be used. The mannitol, especially spray-dried mannitol, can be obtained by a process known from EP 0 896 528 B1. The preparation of the mannitol can be preferably carried out by a process comprising spray-drying and/or fluidised-bed granulation, preferably by a process comprising spray-drying.

Advantageously, mannitol can be used for preparing a pharmaceutical composition of the present invention, especially for the preparation of the mixture (especially compressed mixture) comprising apremilast, mannitol, magnesium stearate and disintegrant, which mannitol is obtainable by a mannitol preparation process comprising:
a) preparing an aqueous mannitol solution, which can optionally comprise a further polyol, e.g. in an amount of up to 2% by weight, based on the total amount of mannitol and further polyol, and
b1) spraying the so obtained solution in an air stream having a temperature between 120 and 300°C, during which the water of the solution is evaporated, or
b2) vortexing the so obtained solution in an air stream having a temperature between 40 and 150° C, during which the water of the solution is evaporated. This mannitol preparation procedure being disclosed in more detail e.g. in EP 0 896 528 B1. The spraying can be for example carried out, by atomisation or disperging by means of nozzles.

Surprisingly, a pharmaceutical composition comprising a mixture, which mixture comprises apremilast, mannitol, magnesium stearate, and disintegrant, wherein mannitol has
- a surface area in the range of 1.0 - 5.0 m²/g, preferably in the range of 2.5 - 4.0 m²/g, more preferably in the range of 2.6 - 3.9 m²/g (e.g. Parteck^{®} M100 or M200), even more preferably in the range of 2.8 - 3.0 m²/g (e.g. Parteck^{®} M200), and/or
- has a pore volume in the range of 0.01 - 0.1 cm³/g, preferably in the range of 0.01 - 0.07 cm³/g (e.g. Parteck^{®} M100 or M200), more preferably in the range of 0.02 - 0.07 cm³/g (e.g. Parteck^{®} M100), show a particularily advantageous storage stability and a low temperature sensitivity, as well as a low humidity sensitivity, as evident from stress condition experiments (e.g. at a temperature of 40°C or 50°C, and 75% relative humidity). Unless explicitly stated otherwise herein, the term surface area refers to BET surface area. A particularily advantageous storage stability and a low temperature sensitivity, as well as a particularily advantageous technological processability has been observed for pharmaceutical compositions comprising a mixture comprising apremilast, and mannitol, in which mixture mannitol has a surface area in the range of 2.6 - 3.9 m²/g, and/or has a pore volume in the range of 0.02 - 0.07 cm³/g.

Studies of binary compacts of the active ingredient with different mannitols have shown that mannitol having a surface area in the range of 1.0 - 5.0 m²/g, preferably in the range of 2.5 - 4.0 m²/g, more preferably in the range of 2.6 - 3.9 m²/g, even more preferably in the range of 2.8 - 3.0 m²/g and a pore volume in the range of 0.01 - 0.1 cm³/g, preferably in the range of 0.01- 0.07 cm³/g, more preferably in the range of 0.02 - 0.07 cm³/g protects very advantageously against the hardening of active ingredient particles, while maintaining an advantageous technological processability.

Advantageously, the pharmaceutical composition of the present invention can comprise a mixture (in particular compressed mixture) comprising apremilast, mannitol, magnesium stearate, and disintegrant, wherein the mannitol is mannitol (especially spray-dried mannitol) having a surface area in the range of 1.0 - 5.0 m²/g, preferably in the range of 2.5 - 4.0 m²/g, more preferably in the range of 2.6 - 3.9 m²/g, even more preferably in the range of 2.8 - 3.0 m²/g. Mannitol having a surface area in these ranges advantageously stabilizes the dissolution rate, especially in tablets exposed to storage and/or stress conditions (e.g. Temperature, humidity).

In particular, the pharmaceutical composition of the present invention can comprise a mixture (in particular compressed mixture) comprising apremilast, mannitol, magnesium stearate, and disintegrant, wherein the mannitol is mannitol (especially spray-dried mannitol) having a pore volume preferably in the range of 0.01 - 0.1 cm³/g, more preferably in the range of 0.01 - 0.07 cm³/g, even more preferably in the range 0.01 - 0.02 cm³/g or in the range 0.02 - 0.07 cm³/g. Mannitol having a pore volume in these ranges provides advantageous compressibility to mixtures subjected to compressing procedures, e.g. during tabletting. The surface area values and surface area range values of mannitol, respectively, indicated in the present application can refer to the mannitol used for preparing the pharmaceutical composition of the present invention, in particular to the mannitol used for preparing the mixture comprising apremilast, mannitol, magnesium stearate and disintegrant. The pore volume values and pore volume range values of mannitol, respectively, in the present application can refer to the mannitol used for preparing the pharmaceutical composition of the present invention, in particular to the mannitol used for preparing the mixture comprising apremilast, mannitol, magnesium stearate and disintegrant.

In particular, the pharmaceutical composition can be or can comprise a mixture (in particular compressed mixture) comprising apremilast, mannitol, magnesium stearate, and disintegrant, which further comprises 0.1 - 45 wt.%, preferably 10 - 35 wt.%, more preferably 15 - 30 wt.% of a filler, based on the total weight of the mixture, said filler being a filler other than a filler comprising or consisting of mannitol. Advantageously, said filler is or comprises microcrystalline cellulose. Microcrystalline cellulose is commercially available, and e.g. described in the Handbook of Pharmaceutical Excipients, 6th edition, ed. by R.C. Rowe et al., 2010 (reprinted).

Filler can be a (preferably solid) filler known in the pharmaceutical art, for example filler selected from dextran, starch, such as corn starch, pregelatinized starch, microcrystalline cellulose, silicified microcrystalline cellulose, xylitol, sucrose, fructose, isomalt, lactitol, maltodextrin, metal salts of phosphoric acid (such as calcium hydrogenphosphate, and combinations thereof.

Furthermore, the pharmaceutical composition of the present invention can comprise a mixture comprising apremilast, mannitol, magnesium stearate, and disintegrant, wherein the disintegrant can be a disintegrant known in the pharmaceutical art, for example disintegrant can be selected from the group of NVP water-swellable polymers, crospovidone, croscarmellose sodium, carmellose sodium or calcium, cellulose derivatives such as low substituted hydroxypropyl cellulose, sodium starch glycolate, alkali and earth alkali salts of carboxymethyl cellulose and ionic resins such as potassium salt of polacrilin and combinations thereof. An "NVP water-swellable polymer" is a water-swellable homo- or heteropolymer containing N-vinylpyrrolidone, e.g. N-vinyl-2-pyrrolidone.

In particular, the pharmaceutical composition of the present invention can comprise a mixture (in particular compressed mixture) comprising apremilast, mannitol, magnesium stearate, and disintegrant, wherein the disintegrant is or comprises croscarmellose sodium; preferably, the disintegrant is croscarmellose sodium. Croscarmellose sodium is commercially available, and e.g. described in the Handbook of Pharmaceutical Excipients, 6th edition, ed. by R.C. Rowe et al., 2010 (reprinted).

Advantageouly, the pharmaceutical composition can be or comprise a mixture comprising apremilast, magnesium stearate, disintegrant, mannitol, which mixture is free of lactose monohydrate, in particular which is free of lactose. In particular, the pharmaceutical composition can be a pharmaceutical composition, which is free of lactose monohydrate, in particular which is free of lactose. Although lactose monohydrate is considered safe for consumption in the amounts present in food and medications, it is also known that lactose-containing medications could cause unpleasant symptoms linked to lactose intolerance and/or allergy. Replacing or reducing the amount of lactose in pharmaceutical compositions is however highly difficult, as lactose provides beneficial properties to pharmaceutical compositions comprising apremilast. In particular, lactose, especially lactose monohydrate, provides a beneficial flowability, and thus processability, to the tableting mixture. For example, a replacement of lactose in pharmaceutical compositions comprising apremilast with microcrystalline cellulose (in absence of mannitol) deteriorates the flowability of the tableting mixture, and consequently the processability of the mixture for tableting. Additionally, for pharmaceutical compositions comprising microcrystalline cellulose (in absence of mannitol), a "coning" occurred in the dissolution analysis, leading to lower profiles. Also, the replacement of lactose with calcium hydrogen phosphate (in absence of mannitol) has not been shown to be advantageous, as this results in very low dissolution profiles and problems with the stability of the formulation (especially an increase in the amount of impurities).

Unexpectedly, after numerous experiments applying various excipient compositions, mannitol has been found as an appropriate substitute for lactose, which even provides an improvement in the flow and other physical (in particular compressibility) properties of the tableting mixture.

Optimization experiments have further shown that using magnesium stearate in a mixture comprising apremilast further improves the flow properties of the mixture. In addition, using magnesium stearate in a mixture to be compressed reduces the likelihood of adherence of the mixture to the faces of tablet press punches. Surprisingly using magnesium stearate in a pharmaceutical composition of the present invention, does not affect the dissolution profiles and thus bioavailability.

In particular, the pharmaceutical composition can be or can comprise a mixture comprising apremilast, mannitol, magnesium stearate and disintegrant, in which mixture the weight ratio between apremilast and magnesium stearate (apremilast : magnesium stearate) is 100:30 or less, especially is in the range from 100:10 to 100:20, in particular is in the range from 100:12 to 100:18. Furthermore, the pharmaceutical composition can be or can comprise a mixture comprising apremilast, mannitol, magnesium stearate and disintegrant, in which mixture the weight ratio between mannitol and magnesium stearate (mannitol : magnesium stearate) is 600:30 or less, especially is in the range from 600:10 to 600:20, in particular is in the range from 600:12 to 600:18. In particular, the pharmaceutical composition can be or can comprise a mixture comprising apremilast, mannitol, magnesium stearate and disintegrant, in which mixture the weight ratio between apremilast and mannitol (apremilast : mannitol) is 1:9 or less, especially is in the range from 1:5 to 1:8, in particular is in the range from 1:5.5 to 1:7.5.

Moreover, the pharmaceutical composition can be or can comprise a mixture, wherein one or more further pharmaceutical excipient(s) are present in the mixture (e.g. compressed mixture) comprising apremilast, mannitol, magnesium stearate, and disintegrant; said one or more further pharmaceutical mixture excipient(s) present in the mixture comprising apremilast, mannitol, magnesium stearate and disintegrant being pharmaceutical excipient(s) which do not comprise (or not consist of) mannitol, and which do not comprise (or not consist of) magnesium stearate. Said one or more further pharmaceutical excipient(s) which are present in the mixture are also referred to hereinafter as one or more further pharmaceutical mixture excipient(s). The terms "pharmaceutical excipient(s)" and "pharmaceutically acceptable excipient(s)" can be used interchangeably herein.

The one or more further pharmaceutical mixture excipient(s) can be e.g. selected from the group of lubricant, glidant, binder, solubility enhancer, flavoring agent, coloring agent, and mixtures thereof. Lubricant (which can be used as further pharmaceutical mixture excipient) can be, for instance, selected from stearic acid; calcium stearate; talc; colloid silica; a wax variety such as beads wax or spermaceti; boric acid; adipic acid, a sulphate such as sodium sulphate; glycol, fumaric acid, stearyl sodium fumarate; sucrose aliphatic acid ester, sodium benzoate, D,L-leucine; a lauryl sulphate such as sodium lauryl sulphate or magnesium lauryl sulphate; silicic anhydride; silicic acid hydrate; starch or a starch derivative, such as corn starch, potato starch, α-starch; dextrin, and mixtures thereof. Glidant (which can be used as further pharmaceutical mixture excipient) can be e.g. selected from talc, colloidal silica, and silicates.

Binder (which can be used as further pharmaceutical mixture excipient) can be, for instance, selected from hydroxypropyl cellulose; hydroxypropyl methyl cellulose; polyvinyl pyrrolidone; polyethylene glycol; glucose; sorbitol; polydextrose; maltodextrin; a starch derivative such as corn starch, potato starch, α-starch or dextrin; a cellulose derivative such as crystalline cellulose; gum arabic; dextran; silicic anhydride; aluminium silicate; calcium silicate; magnesium metasilicate-aluminate; calcium hydrophosphate; calcium carbonate; calcium sulphate, and mixtures thereof.

Solubility enhancer (which can be used as further pharmaceutical mixture excipient) can be, for instance, selected from anionic, cationic, ampholitic or nonionic surfactants, inorganic or organic acids such as (-)-(2S,3S)-tartaric acid, (+)-(2S,3S)-di-O-benzoyltartaric acid, (+)-(2S,3S)-di-O-(4-methylbenzoyl)tartaric acid, (-)-*L*-phenylalanine, benzenesulfonic acid, cyclohexanesulfamic acid, naphthalene-2-sulfonic acid, sebacic acid, camphor-10-sulfonic acid, p-toluenesulfonic acid, ethanesulfonic acid, methanesulfonic acid, adipic acid, pimelic acid, maleic acid, fumaric acid, citric acid, malic acid, succinic acid, glutaric acid, malonic acid, galic acid, ferrulic acid and mixtures thereof.

In a particular advantageous embodiment, the pharmaceutical composition of the present invention is or comprises a mixture which is a compressed mixture, e.g. tablet or granule(s). The pharmaceutical composition comprising a compressed mixture, which is a tablet, can further comprise a coating, especially a film coating, applied on the tablet.

Coating can be prepared employing conventional procedures, e.g. using, for instance, a film coating apparatus. The coating, especially film coating, can be e.g. a sugar coating, a water-soluble film coating, an intestinally soluble film coating. Optionally, pharmaceutical compositions of the present invention can be coated with conventional materials used for coating, in particular film coating, e.g. as described in Pharmaceutical Coating Technology (G. Cole (ed.), 1995). Coating a compressed mixture can for example comprise contacting the compressed mixture with a film coating dispersion to obtain a coated compressed mixture, and optionally drying the coated compressed mixture (e.g. coated tablet or granule). Film coating dispersions can be prepared by combining one or more solvents, such as water or organic solvent(s) such as alcohol(s) (e.g. methanol, ethanol, isopropanol), ketones (e.g. acetone), and mixtures thereof, with film-coating excipient(s).

A coating can be a coating comprising a polymer e.g. selected from cellulose ethers such as hyprolose or hypromelose, block copolymer of polyvinyl alcohol and polyethylene glycol (commercially obtainable under trade name of Kollicoat IR or Kollicoat protect), polyvinyl alcohol, aminoalkyl methacrylate copolymers (such as Eudragit EPO), methacrylic acid copolymers, salts of carboxymethylcellulose, and mixtures thereof. Said coating can further comprise coating additive(s) selected from plasticizers, pigments, colorants, opacifying agents, preservatives, antitacking agents and mixtures thereof. The thickness of the coating (in particular film coating) applied on the tablet can be in the range of from 10 to 100 µm, preferably from 15 to 50 µm.

The coating of the tablet (especially film coating), where present, can include from 10% to 95% of polymer, based on the weight of the coating. The coating (especially film coating) of the tablet, where present, can comprise from 20% to 90% of polymer, optionally from 2% to 30% by weight of plasticizer, each based on the weight of the coating, optionally glidant, optionally opacifying agent, optionally colorant, and optionally a glidant. In an embodiment, the film coating of the tablet, where present, can comprise from 20% to 90% of polyvinyl alcohol (PVA), from 2% to 30% by weight of plasticizer, each based on the weight of the coating, preferably the plasticizer being or including polyethylene glycol; the film coating can further comprise optionally glidant, optionally opacifying agent, optionally colorant, and optionally a glidant.

Advantageous examples of polymer for the coating include, but are not limited to, hydroxypropyl methylcellulose, polyvinyl alcohol (PVA), ethyl cellulose, methacrylic polymers, hydroxypropyl cellulose, starch, and mixtures thereof. The coating layer polymer can be preferably PVA. Presence of PVA in the coating allows for enhanced film adhesion, and facilitates faster coating of the drug.

Plasticizers can be selected from, but are not limited to, triacetin, diethyl phthalate, tributyl sebacate, polyethylene glycol (PEG), glycerin, triacetin, propylene glycol, polypropylene glycol, glycerol, sorbitol, acetyl triethyl citrate, and mixtures thereof. The plasticizer can be advantageously polyethylene glycol. The coating can also optionally comprise a glidant such as talc, fumed silica, magnesium stearate, or a combination thereof. The coating can also optionally comprise an opacifying agent, such as titanium dioxide. The coating layer may optionally comprise one or more colorants, for example, iron oxide based colorant(s). Examples of commercially available coating material include Opadry^{®} HP, Opadry^{®} II HP white.

The pharmaceutical compositions provided herein exhibit advantageous physical and/or pharmacological properties. Such properties include, but are not limited to, low friability, content uniformity, flow properties for manufacture, advantageous dissolution and bioavailability, and/or storage stability.

A pharmaceutical composition (especially in the form of a tablet or granule(s)) of the present invention can be prepared by any of the method known in the art. For example, the compositions can be prepared by uniformly admixing the active ingredient with excipients, and then, if necessary, shaping the product into the desired presentation (e.g., compaction such as roller- compaction). If desired, tablets can be coated by standard aqueous or non-aqueous techniques.

In particular, the pharmaceutical composition of the present invention can be prepared by a preparation method, preferably a solvent free preparation method, which can be a direct compression method or a (preferably solvent free) granulation method such as roller compaction, or slugging; the direct compression method being preferred.

The "direct compression method" is a method of formulation wherein a mixture comprising apremilast and excipients is subjected to direct compression moulding, in particular without any preagglomeration processes.

The "dry granulation method" is a method which comprises that a mixture comprising apremilast and excipients is subjected to compression moulding into comprimate or sheet, wherein granules are produced by crushing and division of comprimates and/or sheets by a suitable method. Such methods are described in The Theory and Practice of Industrial Pharmacy (Third Edition)(Leon Lachman et al., LEA & FEBIGER 1986) or Pharmaceutical Dosage Forms: Tablets Volume 1 (Second Edition)(Herbert A. Lieberman et al.: MARCEL DEKKER Inc. 1989).

Granules can be filled into a capsule to prepare an encapsulated product or, after an optional addition of pharmaceutical additives selected from disintegrating agents and/or lubricants and/or other additives, formulated into tablets by means of compression moulding, e.g. in a tableting machine.

The amount of apremilast in a pharmaceutical composition of the invention may vary in function of the conditions such as the activity of the medicine, the disease, age and body weight of the patient. The amount of apremilast in a pharmaceutical composition of the invention (e.g. in a tablet or in a pharmaceutical composition comprising granule(s)) can be in the range of from 60 to 10 mg, especially in the range of from 30 to 10 mg.

The pharmaceutical composition of the present invention may be in any form such as, for example, in the form of a tablet, capsules, pellets, granulate. The pharmaceutical composition is preferably suitable for oral administration. Preferably, the pharmaceutical composition can be an oral pharmaceutical composition, especially an oral tablet, most preferably oral film-coated tablet.

According to a further aspect of the present invention, there is provided a process for preparing a pharmaceutical composition, especially a pharmaceutical composition of the present invention as described herein above, said process comprising:
a) preparing a mixture, said mixture comprising:
   3-20 wt.%, preferably 5 - 15 wt.%, more preferably 7.5 - 12.5 wt.% of apremilast, based on the total weight of said mixture (prepared in step a)),
   40-90 wt.%, preferably 50 - 80 wt.%, more preferably 55 - 75 wt.% of mannitol, based on the total weight of said mixture (prepared in step a)),
   0.5 - 3 wt.%, preferably 1 - 2 wt.%, more preferably 1.2 - 1.8 wt.% of magnesium stearate,, based on the total weight of said mixture (prepared in step a)), and
   0.5 - 6 wt.%, preferably 1 - 5 wt.%, more preferably 2 - 4 wt.% of a disintegrant, based on the total weight of said mixture (prepared in step a)), and optionally comprising further pharmaceutically acceptable excipient(s),
b) compressing said mixture to obtain a compressed mixture, in particular tablet,
c) optionally coating the compressed mixture, in particular tablet, with a coating, in particular a film coating.

The mixture comprising apremilast, mannitol, magnesium stearate, and disintegrant, has been described supra in more detail.

Step a) of preparing a mixture can comprise:
i) mixing (in particular triturating) apremilast with a portion of filler (especially microcrystalline cellulose) and optionally sieving the so obtained apremilast containing mixture,
ii) mixing the so obtained optionally sieved apremilast containing mixture (in particular triturate) with mannitol, disintegrant and optional further pharmaceutically acceptable excipient(s),
iii) adding the magnesium stearate to the mixture obtained in step ii),
iv) mixing of the mixture obtained in step iii), or
step a) of preparing a mixture can comprise:
I.) mixing apremilast and all excipients except magnesium stearate,
II.) sieving the mixture obtained in step I.),
III.) optionally mixing the sieved mixture obtained in step II.),
IV.) addition of magnesium stearate to the mixture obtained in step II.) or in step III.),
V.) mixing the mixture obtained in step IV.).

Step b) can be in particular compressing (especially tableting) on a tablet press.

According to a further aspect of the present invention, there is provided a method for crystallizing apremilast, said method comprising the following steps:
a) preparing a solution comprising apremilast and a solvent, said solvent being or comprising acetone,
b) optionally adding seeding material to the solution comprising apremilast and a solvent to obtain a seeding material containing mixture,
c) adding an antisolvent (optionally said antisolvent having a temperature in the temperature range of from 35°C to 55°C, preferably from 40°C to 50°C), and optionally adding seeding material to the solution comprising apremilast prepared in step a) or to the seeding crystal containing mixture optionally prepared in step b),
   optionally said solution comprising apremilast prepared in step a) or said seeding crystal containing mixture optionally prepared in step b) having a temperature in the temperature range of from 35°C to 55°C, preferably from 40°C to 50°C,
   during an antisolvent addition time period of from 1 minute to 20 hours, preferably of from 10 minutes to 1 hour, to obtain a mixture comprising apremilast, solvent, and antisolvent, said antisolvent being or comprising ethanol,
   optionally said mixture comprising apremilast, solvent, and antisolvent obtained in step c) having a temperature in the temperature range of from 35°C to 55°C, preferably from 40°C to 50°C,
d) optionally increasing the temperature of the mixture comprising apremilast, solvent, and antisolvent obtained in step c) to a temperature in the temperature range up to 65°C, and optionally adjusting (in particular decreasing) the temperature of the mixture comprising apremilast, solvent, and antisolvent to a temperature in the temperature range of from 35°C to 55°C, preferably from 40°C to 50°C,
e) subjecting the mixture comprising apremilast, solvent, and antisolvent to agitation at a temperature in a first crystallization temperature range of from 35 °C to 55 °C, preferably from 40°C to 50°C, during a first crystallization period of from 5 hours to 25 hours, preferably of from 10 hours to 20 hours, to obtain a suspension comprising apremilast,
f) decreasing the temperature of the suspension comprising apremilast to a temperature in a second crystallization temperature range of from -20°C to 20°C, preferably of from -10°C to 10°C,
g) separating crystalline apremilast from the suspension comprising apremilast,
h) optionally drying the separated crystalline apremilast.

The present inventors carried out numerous experiments for studying and developing crystallization processes of the active ingredient apremilast. Surprisingly, the present inventors found that by controlling the crystallization parameters (in particular temperature, time), apremilast with advantageous physicochemical properties can be prepared. In particular, apremilast obtainable by a crystallization process of the present invention allows to achieve an advantageous stress stability of the dissolution rate of apremilast contained in pharmaceutical compositions. In particular, during the development of the process starting from the solvent system comprising or consisting of acetone and ethanol, the present inventors found that within the crystallization parameters commonly used in crystallization processes where antisolvent is added, apremilast unexpectedly shows a tendency to form particles in needle form, especially small thin needle like primary particles. These apremilast particles in needle form can be present in the bulk material as separate fraction, or as adhered fraction on bigger particles or in the form of spherical agglomerates. During the development of an apremilast formulation with mannitol, the present inventors unexpectedly found that pharmaceutical compositions comprising apremilast obtainable by a crystallization process of the present invention allows to achieve advantageous dissolution profiles, which have no or merely have a very low sensitivity as regards to changes in formulation parameters and as regards to storage and stress conditions (temperature, humidity). In particular, apremilast obtainable by a crystallization process of the present invention provides improved flow properties and an improved dissolution stability under stress conditions. The dissolution profile of apremilast obtainable by a crystallization process of the present invention remains similar before and after exposure to stress (e.g. stress due to exposure to a temperature e.g. above 25°C, and/or relative humidity e.g. 75% or more). Apremilast obtainable by the crystallization process of the present invention therefore allows the preparation of particularily advantageous pharmaceutical compositions comprising apremilast. Without wishing to be bound by theory, it is assumed that compositions comprising considerable amounts of apremilast in needle form have less advantageous properties, due to an disadvantageous crushing of apremilast agglomerates and thin apremilast primary particles during the preparation of the pharmaceutical composition.

Advantagously, step a) of preparing a solution comprising apremilast and a solvent, said solvent being or comprising acetone, can comprise heating the solution apremilast and a solvent to the reflux temperature of the solvent or a temperature in the range from 6°C below reflux temperature of the solvent to the reflux temperature of the solvent. In particular, step a) of preparing a solution comprising apremilast and a solvent can comprise heating the solution apremilast and a solvent to a temperature of at least 50°C, e.g. to a temperature in the range from 50°C to 56°C.

Step a) can comprise adding apremilast in one or more portions to the solvent for preparing a solution comprising apremilast and a solvent. During step a) of preparing a solution comprising apremilast and a solvent, the solvent can be subjected to agitation, for example stirring. An addition of seeding material may facilitate the crystallization process. An addition of seeding material can be optionally carried out before the adding of an antisolvent, or during step c) of adding an antisolvent, or both before adding of an antisolvent and during step c) of adding an antisolvent.

Step a) of preparing a solution comprising apremilast and a solvent can be preparing a solution comprising apremilast and a solvent, said solvent being or comprising at least 98 wt.% of acetone, especially 99.8 to 100 wt.% of acetone, based on the total weight of said solvent. In a preferred embodiment, said solvent being free of water or comprising not more than 2 wt.% of water, especially less than 0.2 wt.% of water, each based on the total weight of said solvent.

After having prepared a solution comprising apremilast and a solvent, optionally seeding material can be added to the solution comprising apremilast and a solvent to obtain a seeding material containing mixture. The seeding material can be added in one or more portions. Advantageously, the seeding material can be added to a solution comprising apremilast and a solvent having a temperature in the temperature range of from 35°C to 55°C, preferably from 40°C to 50°C. The temperature of the seeding material containing mixture can be subsequently adjusted e.g. to a temperature in the range of from 35°C to 55°C, preferably from 40°C to 50°C, until step c) is started.

Step c) of the method for crystallizing apremilast is or comprises: adding an antisolvent and optionally adding seeding material to the solution comprising apremilast prepared in step a) or to the seeding crystal containing mixture optionally prepared in step b) during an antisolvent addition time period of from 1 minute to 20 hours, preferably of from 10 minutes to 1 hour, to obtain a mixture comprising apremilast, solvent, and antisolvent, said antisolvent being or comprising ethanol.

Advantageously, in step c) said solution comprising apremilast prepared in step a) or said seeding crystal containing mixture optionally prepared in step b) can have a temperature in the temperature range of from 35°C to 55°C, preferably from 40°C to 50°C. In step c), during the antisolvent addition time period, the temperature of the mixture comprising apremilast, solvent, and antisolvent (which mixture is obtained after having started the addition of antisolvent) can have a temperature in the temperature range of from 35°C to 55°C, preferably from 40°C to 50°C.

In particular, in step c) said solution comprising apremilast prepared in step a) or said seeding crystal containing mixture optionally prepared in step b) can have a temperature in the temperature range of from 35°C to 55°C, preferably from 40°C to 50°C, and during the antisolvent addition time period, the temperature of the mixture comprising apremilast, solvent, and antisolvent can have a temperature in the temperature range of from 35°C to 55°C, preferably from 40°C to 50°C. Optionally, in step c) said antisolvent can have a temperature in the temperature range of from 35°C to 55°C, preferably from 40°C to 50°C. Furthermore, it has been found to be advantagous if both (A) said antisolvent has temperature in the temperature range of from 35°C to 55°C, preferably from 40°C to 50°C, and (B) said solution comprising apremilast prepared in step a) or said seeding crystal containing mixture optionally prepared in step b) has a temperature in the temperature range of from 35°C to 55°C, preferably from 40°C to 50°C. Especially, it has been found to be advantagous if both (A) said antisolvent has temperature in the temperature range of from 40°C to 50°C, and (B) said solution comprising apremilast prepared in step a) or said seeding crystal containing mixture optionally prepared in step b) has a temperature in the temperature range of from 40°C to 50°C.

Said antisolvent can be or can comprise ethanol. Advantageously, the antisolvent is ethanol. Preferably, said antisolvent is ethanol or comprises at least 96 wt.% of ethanol, especially 98 to 100 wt.% of ethanol, based on the total weight of said antisolvent. In a preferred embodiment, said solvent being free of water or comprising less than 4 wt.% of water, especially less than 2 wt.% of water, each based on the total weight of said antisolvent.

In particular, in step c) the antisolvent can be added in an amount that the mixture comprising apremilast, solvent and antisolvent (which is obtained in step c) and/or subjected to agitation in step e)) comprises ethanol and acetone in a ratio ethanol to acetone (ethanol : acetone) which can be in the range of from 1:5 to 5: 1, preferably of from 1: 3 to 3: 1, more preferably in an amount of 1:2 to 2:1, especially in the range of from 2:1 to 5:1, further especially in the range of from 2:1 to 3:1. Furthermore, the mixture comprising apremilast, solvent and antisolvent subjected to agitation in step e) can comprise ethanol and acetone in a ratio ethanol to acetone (ethanol : acetone) which can be in the range of from 1:5 to 5: 1, preferably of from 1:3 to 3:1, more preferably in an amount of 1:2 to 2:1, especially in the range of from 2:1 to 5:1, further especially in the range of from 2:1 to 3:1.

Advantageously, step c) of the method for crystallizing apremilast is or comprises both adding an antisolvent and adding seeding material.

When adding an antisolvent and optionally adding seeding material to the solution comprising apremilast prepared in step a) or to the seeding crystal containing mixture optionally prepared in step b), the solution comprising apremilast prepared in step a), or the seeding crystal containing mixture optionally prepared in step b), can be subjected to agitation, such as subjected to stirring.

Optionally, in step d) the temperature of the mixture comprising apremilast, solvent, and antisolvent obtained in step c) can be increased to a temperature in the temperature range up to 65°C. Furthermore, an optional adjusting (in particular a decreasing of the temperature of the mixture comprising apremilast, solvent, and antisolvent, especially after having increased the temperature of the mixture comprising apremilast, solvent, and antisolvent obtained in step c) to a temperature in the temperature range up to 65°C) of the temperature of the mixture comprising apremilast, solvent, and antisolvent to a temperature in the temperature range of from 35°C to 55°C, preferably from 40°C to 50°C can be carried out.

In particular, step e) of subjecting the mixture comprising apremilast, solvent, and antisolvent to agitation at a temperature in a first crystallization temperature range can be carried out directly after step c) of adding an antisolvent and optionally adding seeding material (i.e. without optional step d)).

Especially, the mixture comprising apremilast, solvent, and antisolvent obtained in step c) can have a temperature in the temperature range of from 35°C to 55°C, preferably from 40°C to 50°C, and can be kept at a temperature in the temperature range of from 35°C to 55°C, preferably from 40°C to 50°C, until step e) begins.

The crystallization method of the present invention further comprises the step of subjecting the mixture comprising apremilast, solvent, and antisolvent to agitation at a temperature in a first crystallization temperature range of from 35 °C to 55 °C, preferably from 40°C to 50°C, during a first crystallization period of from 5 hours to 25 hours, preferably of from 10 hours to 20 hours, to obtain a suspension comprising apremilast. Subjecting the mixture to agitation can be carried out according to the general knowledge in the art, and can be or comprise stirring of the mixture.

Subsequently, the temperature of the suspension comprising apremilast (which can have a temperature in a first crystallization temperature range of from 35 °C to 55 °C, preferably from 40°C to 50°C), is decreased to a temperature in a second crystallization temperature range of from -20°C to 20°C, preferably of from -10°C to 10°C. After having decreased the temperature to a temperature in a second crystallization temperature range of from -20°C to 20°C, preferably of from -10°C to 10°C, the suspension can be kept at the crystallization temperature range of from -20°C to 20°C, preferably of from -10°C to 10°C. When decreasing the temperature of the suspension comprising apremilast to a temperature in a second crystallization temperature range, the suspension comprising apremilast can be subjected to agitation, such as subjected to stirring, or can be not subjected to agitation, e.g. not subjected to stirring. In particular, the decreasing of the temperature to a temperature in a second crystallization temperature range can be carried out directly after the end of the first crystallization period. Without wishing to be bound by theory, decreasing the temperature to a temperature in a second crystallization temperature range of from -20°C to 20°C, preferably of from - 10°C to 10°C, can increase the yield of crystalline apremilast.

In a subsequent step, crystalline apremilast is separated from the suspension comprising apremilast. Separating the crystalline apremilast can be carried out according to the general knowledge in the art, e.g. by subjecting the suspension comprising apremilast to filtration. The drying can be e.g. carried out by heating the crystalline apremilast to a temperature in the temperature range up to (and including) 40°C, and/or by subjecting the crystalline apremilast to a pressure below ambient pressure (ambient pressure can be in particular a pressure of 1 atm), e.g. to a pressure of less than 100 mbar, preferably less than 50 mbar, e.g. in the range of 50 mbar to less than 100 mbar Pa.

The term "seeding material" comprises both seeding crystals of apremilast and silica particles (which can be especially used for an intial crystallization of apremilast); preferably, seeding material is apremilast crystal(s). In particular, seeding crystals of apremilast can be obtained from a crystallization solvent mixture (comprising the compound to be crystallized) by slow evaporation under stirring, in particular followed by cooling heating cycling. Solution can be optionally inoculated by silica particles to initiate crystallization. Optionally, oily residue after reaction can be purified by flash chromatography before preparation of first seeding crystals. So obtained crystals can be used for inoculation in crystallization of apremilast to obtain pure seeding material. Pure material as obtained by routine crystallization or manufacture can be used for seeding. In particular, apremilast seeding crystals (e.g. crystals of apremilast Form B) can be prepared according to a preparation method disclosed in WO 03/080049, WO 2009/120167.

Apremilast, when crystallized from acetone/ethanol solvent system, tends to crystallize in the form of needle like thin particles. If considerable amounts of needle like thin particles are present in apremilast bulk material obtained after crystallization, such material will show poorer flow properties, as well as a reduced dissolution after exposure to temperature stress conditions and/or humidity stress conditions (40 °C /75 % RH (relative humidity)). The dissolution profile of tablets comprising considerable amounts of apremilast in needle form can show a decreased dissolution, after exposure to stress stability test conditions (40 °C /75 % RH). Apremilast particles in needle form can be present in the apremilast bulk material as separate fraction, or as adhered fraction on bigger particles, or in the form of agglomerates.

The present inventors surprisingly found that a careful control of process parameters during the crystallization, including e.g. temperature of crystallization, crystallization time at a particular temperature range, crystallize apremilast that is substantially free of needle like thin particles can be obtained.

According to a further aspect of the present invention, there is provided apremilast (especially crystalline apremilast) obtainable by the method for crystalizing apremilast of the present invention. In particular, there is provided apremilast (especially crystalline apremilast) obtainable by the method for crystallizing apremilast comprising:
a) preparing a solution comprising apremilast and a solvent, said solvent being or comprising acetone,
b) optionally adding seeding material to the solution comprising apremilast and a solvent to obtain a seeding material containing mixture,
c) adding an antisolvent and optionally adding seeding material to the solution comprising apremilast prepared in step a) or to the seeding crystal containing mixture optionally prepared in step b) during an antisolvent addition time period of from 1 minute to 20 hours, preferably of from 10 minutes to 1 hour, to obtain a mixture comprising apremilast, solvent, and antisolvent,
   said antisolvent being or comprising ethanol,
d) optionally increasing the temperature of the mixture comprising apremilast, solvent, and antisolvent obtained in step c) to a temperature in the temperature range up to 65°C,
e) subjecting the mixture comprising apremilast, solvent, and antisolvent to agitation at a temperature in a first crystallization temperature range of from 35 °C to 55 °C, preferably from 40°C to 50°C, during a first crystallization period of from 5 hours to 25 hours, preferably of from 10 hours to 20 hours, to obtain a suspension comprising apremilast,
f) decreasing the temperature of the suspension comprising apremilast to a temperature in a second crystallization temperature range of from -20°C to 20°C, preferably of from -10°C to 10°C,
g) separating crystalline apremilast from the suspension comprising apremilast,
h) optionally drying the separated crystalline apremilast.

Especially, there is provided apremilast obtainable by the method for crystallizing apremilast of the present invention, which is substantially free of apremilast particles in needle form.

The apremilast particle size can be determined from SEM images.The width of the apremilast particles described in the comperative examples can be e.g. 1.7 or 1.6 or 2.4 µm, and the height of particles can be e.g. 0.6 or 0.7 µm. The particles of apremilast obtained by the crystallization of the present invention are thicker, the width of the particles can be e.g. 7.6 or 6.7 or 11.5 or 29 or 8.7 µm, and height can be e.g. 3.3 or 2.5 or 7.0 or 7,7 or 2,6 µm. Therefore, for apremilast particles obtainable by the method for crystallizing apremilast of the present invention, the preferred particle width can be not less than 5.0 µm, preferably not less than 6 µm, more preferably not less than 8 µm, most preferably not less than 10 µm, e.g. in the range 5.0 to 10 µm, and preferred particles height can be not less than 2.0 µm, preferably not less than 3 µm, more preferably not less than 5 µm, most preferably not less than 7µm, e.g. in the range 2.0 to 7 µm. In an embodiment, particle width and particle height can be average particle width and average particle height, which can be calculated based on particles number average (which can be e.g. determined from SEM images using a computer program, e.g. as described in the section entitled "Analytical methods" below).

Preferably, "substantially free of apremilast particles in needle form" can have the meaning free of particles in needle form or containing less than 10 percent by weight, preferably less than 5 percent by weight, and more preferably less than 2 percent of particles in needle form.

Furthermore, "substantially free of apremilast particles in needle form" can have the meaning free of particles in needle form or containing less than 10 volume percent, preferably less than 5 volume percent, and more preferably less than 2 volume percent of particles in needle form.

In a preferred embodiment of the pharmaceutical composition, the pharmaceutical composition comprises apremilast which is or comprises apremilast obtainable by the process for preparing apremilast according to the present invention. Apremilast obtainable by the process for preparing apremilast according to the present invention provides the advantage of avoiding using thin needle like primary particles as separate fraction or in the form of spherical agglomerates. At the same time, by designing the desired morphology of apremilast in the crystallization process, grinding of the active substance can be avoided. This is highly advantageous as apremilast comprising agglomerates and/or needle like primary particles makes it difficult to adjust the particle size by grinding. Apremilast obtainable by the process for preparing apremilast according to the present invention therefore provides the advantage of providing apremilast having a desired particle size, as well as providing an advantageous dissolution rate in the final product (in particular pharmaceutical composition of the invention) without requiring a grinding procedure, as well as providing an advantageous dissolution rate in the final product (in particular pharmaceutical composition of the invention) even after exposure to stress conditions (temperature stress and/or relative humidity stress), as well as provides desired physicochemical properties, such as the resistance of the particles to mechanical stress. This ensures that the particles do not or essentially not crumble during the manufacturing process (especially during a compression step) of the pharmaceutical compositions of the present invention, especially film-coated tablets. The present inventors surprisingly found that replacing the apremilast containing agglomerates and brittle needle like particles with one containing thicker primary particles of appropriate size, contributes to achieving an advantageous dissolution rate, even after exposure to stress conditions (temperature stress and/or relative humidity stress), as well as contributes to providing an advantageous stability of dissolving rate of the pharmaceutical composition comprising apremilast, even when said composition is stored under accelerated stability conditions.

Pharmaceutical compositions comprising apremilast obtainable according to the crystallization process of the present invention are therefore pharmaceutical compositions having a highly advantageous dissolution profile and dissolution profile stability, even after having subjected the pharmaceutical composition to storage at elevated temperature and/or humidity (as simulated under accelerated/stress conditions). Pharmaceutical compositions of the present invention, wherein needle-like particles of apremilast are present, are surprisingly not problematic for flow or other physical properties due to the presence of mannitol (and the further excipients of the pharmaceutical composition of the present invention). Pharmaceutical compositions of the present invention, wherein no or substantially no or less apremilast particles having needle-form are present, have physical properties which are still further improved.

Apremilast obtainable according to the present invention and pharmaceutical compositions of the present invention can be for use as a medicament. Apremilast obtainable according to the present invention and pharmaceutical compositions of the present invention can be for use in a method of treating or preventing diseases or disorders including, but not limited to, those related to PDE4, TNFα, cAMP and/or angiogenesis, and can be in particular for use in treating or preventing diseases or disorders, such as inflammatory diseases, pulmonary diseases, autoimmune diseases and immunological diseases; said diseases and disorders can be for example selected from Behçet's disease, psoriasis (especially plaque psoriasis), Crohn's disease, and arthritis other than rheumatoid arthritis and combinations thereof.

The following examples illustrate the invention and are not intended to restrict the scope of the invention in any way.

### Examples

### Examples 1 to 9 (according to the present invention) and Reference Examples 1 and 2

Table 1 presents a reference composition 1 prepared using the Core Formulation of Table 1 of Example 5.1. and Coating Formulation 1 of Example 5.2.1 of WO 2013/101810 A1 . This reference composition is considered to be identical with or similar to the commercially available Otezla^{®} tablet.

**Table 1: Composition of the reference composition 1 (tablet)**

| **Location of ingredients** | **Ingredient type** | **Ingredient** | **Amount [%]** |
|---|---|---|---|
| Core | | Active ingredient A (apremilast) | 10.00% |
| | 316, Fast-Flo^{®} | Lactose monohydrate | 60.00% |
| | Avicel^{®} PH-102 | Microcrystalline cellulose | 26.25% |
| | Ac-Di-Sol^{®} | Croscarmellose Sodium | 3.00% |
| | / | Magnesium stearate | 0.75 % |
| | *Total core* | | 100.0% |
| Coating (beige)^{∗} | / | Polyvinyl alcohol | 40.0% |
| | | Macrogol/PEG 3350 | 20.2% |
| | | Talc | 14.8% |
| | | Titanium dioxide | 22.99% |
| | | Red iron oxide | 1.18% |
| | | Yellow iron oxide | 0.43% |
| | | Black iron oxide | 0.4% |
| | *Total coating* | | 100.0% |

| | | | |
|---|---|---|---|
| ^{∗} Three coating formulations are presented in Example 5.2.1 of WO 2013/101810 A1. | | | |

Table 2 shows examples of pharmaceutical compositions according to the present invention. The pharmaceutical compositions shown in Table 2 comprise 30 mg of apremilast.

Example 6 comprises apremilast prepared according to the inventive method for crystallizing apremilast disclosed herein.

**Table 2: Examples of the composition of 30 mg dose formulations with apremilast of the invention.**

| | | **Examples 1 and 2** | | **Example 3** | | **Example 4** | | **Examples 5 and 6** | | **Example 7** | | **Example 8** | | **Example 9** | |
|---|---|---|---|---|---|---|---|---|---|---|---|---|---|---|---|
| | | **Amount per unit** | | | | | | | | | | | | | |
| **Formulation** | **Material** | **[mg]** | **[%]** | **[mg]** | **[%]** | **[mg]** | **[%]** | **[mg]** | **[%]** | **[mg]** | **[%]** | **[mg]** | **[%]** | **[mg]** | **[%]** |
| *Core* | Apremilast | 30.00 | 10.00 | 30.00 | 10.00 | 30.00 | 10.00 | 30.00 | 10.00 | 30.00 | 10.00 | 30.00 | 10.00 | 20.00 | 10.00 |
| | Microcrystalline cellulose (Type 102) | 78.75 | 26.25 | 78.75 | 26.25 | 78.75 | 26.25 | 63.75 | 21.25 | 90.75 | 30.25 | 44.85 | 14.95 | 42.50 | 21.25 |
| | Croscarmellose Sodium (Ac-Di-Sol^{®}) | 9.00 | 3.00 | 9.00 | 3.00 | 9.00 | 3.00 | 9.00 | 3.00 | 9.00 | 3.00 | 9.00 | 3.00 | 6.00 | 3.00 |
| | Magnesium stearate | 2.25 | 0.75 | 4.50 | 1.50 | 4.50 | 1.50 | 4.50 | 1.50 | 4.50 | 1.50 | 5.4 | 1.80 | 3.00 | 1.50 |
| | Mannitol (Parteck^{®} M200) | 180.00 | 60.00 | 177.75 | 59.25 | / | / | / | / | / | / | / | / | / | / |
| | Mannitol (Parteck^{®} M100) | / | / | / | / | 177.75 | 59.25 | 192.75 | 64.25 | / | / | / | / | 128.50 | 64.25 |
| | Mannitol (Pearlitol^{®} 100 SD) | / | / | / | / | / | / | / | / | 165.75 | 55.25 | / | / | / | / |
| | Mannitol (Mannogem^{®} EZ) | / | / | / | / | / | / | / | / | / | / | 210.75 | 70.25 | / | / |
| | **Total core** | **300.00** | 100.00 | **300.00** | 100.00 | **300.00** | 100.00 | **300.00** | 100.00 | **300.00** | 100.00 | **300.00** | 100.00 | **200.00** | 100.00 |
| *Film **coating*** | Opadry White | 11.76 | 98.00 | 11.76 | 98.00 | 11.76 | 98.00 | 11.76 | 98.00 | 11.76 | 98.00 | 11.76 | 98.00 | 6.97 | 87.13 |
| | Red iron oxide | 0.14 | 1.16 | 0.14 | 1.16 | 0.14 | 1.16 | 0.14 | 1.16 | 0.14 | 1.16 | 0.14 | 1.16 | 0.10 | 1.25 |
| | Yellow iron oxide | 0.05 | 0.42 | 0.05 | 0.42 | 0.0 | 0.42 | 0.05 | 0.42 | 0.05 | 0.42 | 0.05 | 0.42 | 0.93 | 11.62 |
| | Black iron oxide E80 | 0.05 | 0.42 | 0.05 | 0.42 | 0.05 | 0.42 | 0.05 | 0.42 | 0.05 | 0.42 | 0.05 | 0.42 | / | / |
| | **Total coating** | **12.00** | 100.00 | **12.00** | 100.00 | **12.00** | 100.00 | **12.00** | 100.00 | **12.00** | 100.00 | **12.00** | 100.00 | **8.00** | 100.00 |
| **Total tablet** | | **312.00** | | **312.00** | | **312.00** | | **312.00** | | | **312.00** | | **312.00** | **208.00** | |

The process of making pharmaceutical compositions comprising apremilast (reference and inventive examples) is as follows:
1. Preparing a tableting mixture comprising active ingredient and core excipients (as indicated in Table 2: apremilast, mannitol, magnesium stearate, croscarmellose sodium (croscarmellose sodium being a disintegrant), microcrystalline cellulose (microcrystalline cellulose being a filler)).
2. The tableting mixture is tableted on a tablet press.
3. Coating suspension is prepared and the cores are film coated.

Two variants for making pharmaceutical compositions comprising apremilast (reference and inventive examples) have been carried out:

### Variant A

A-1. Apremilast is mixed (triturated) with a portion of microcrystalline cellulose and sieved. Apremilast triturate is mixed with the remaining, optionally sieved raw materials without magnesium stearate in the container. After adding the magnesium stearate, the final mixing or lubrication, respectively, of the tableting mixture is performed.

A-2. The tableting mixture is tableted on a tablet press.

A-3. Coating suspension is prepared and the cores are film coated.

### Variant B

β-1.Premixing of all raw materials (except magnesium stearate) in a container and sieving is performed. Mixing and then, after the addition of magnesium stearate, the final mixing or lubrication, respectively, is performed.

B-2. The tableting mixture is tableted on a tablet press.

B-3. Finally, coating suspension is prepared and the cores are film coated.

Table 3 show the ingredients used for preparing Reference Examples 1 and 2. Reference Examples 1 and 2 provide compositions representative for compositions according to Table 1 of WO 2013/101810 A1.

**Table 3**

| | | **Reference Example** 1 **and 2** | |
|---|---|---|---|
| | | **Amount per unit** | |
| **Formulation** | **Material** | **[mg]** | **[%]** |
| ***Core*** | Apremilast | 30.00 | 10.00 |
| | Microcrystalline cellulose (Type 102) | 78.75 | 26.25 |
| | Croscarmellose Sodium (Ac-Di-Sol) | 9.00 | 3.00 |
| | Magnesium stearate | 2.25 | 0.75 |
| | Lactose monohydrate, Spray dried (Flowlac 100) | 180.00 | 60.00 |
| | **Total core** | **300.00** | 100.00 |
| ***Film coating*** | Opadry White | 11.76 | 98.00 |
| | Red iron oxide | 0.14 | 1.16 |
| | Yellow iron oxide | 0.05 | 0.42 |
| | Black iron oxide E80 | 0.05 | 0.42 |
| | **Total coating** | **12.00** | 100.00 |
| **Total tablet** | | **312.00** | |

Table 4 shows the physical properties of the tableting mixture, the tableting parameters and the properties of the cores (compressed mixtures) and film-coated tablets of reference and inventive examples, respectively.

**Table 4**

| | Reference Example 1 | Reference Example 2 | Example 1 | Example 5 | Example 9 |
|---|---|---|---|---|---|
| Crystallization process of Apremilast | Unknown crystallization procedure | Unknown crystallization procedure | Unknown crystallization procedure | Prior art crystallization | Crystallization according to invention |
| ***Tableting mixture*** | | | | | |
| Hausner ratio | 1.36 | 1.39 | 1.32 | No data | 1.24 |
| Flow time [s] | no flow | no data | 40.1 | 31.7 | 35.4 |
| ***Tableting*** | | | | | |
| Ratio of tablet strength and main pressure at compression | 10.4 | 11.4 | no data | 15.3 | 15.6 |
| ***Film coated tablets*** | | | | | |
| Uniformity of dosage units, acceptance value (AV) | 3.7 % | 5.4 % | no data | 2.6 % | 2.4 % |
| Decrease of strength of film coated tablets at accelerated stress stability conditions | no data | 27.5 % | 10% | No data | no data |

A comparison of the physical properties of the mixtures shows much better flow properties of Example 1 versus Reference Examples 1 and 2 (lower Hausner ratio and shorter flow time). By increasing the amount of Magnesium stearate, these properties are further improved (Example 5 as compared to Example 1). Example 9 using apremilast crystallized according to the invention has a highly advantageous flow time and hence highly advantageous flow properties. Examples 5 and 9 also show much better compressibility (higher ratios of tablet strength and main pressure) compared to the Reference Examples, which in the industrial environment allows faster tableting, lower brittleness and reduced wear of the tableting punches. The improvement in the flowability of the mixture is also reflected in the lower fluctuation of the uniformity of the content in the film-coated tablets of Examples 5 and 9, when compared to Reference Examples 1 and 2 (lower AV values). The decrease in the strength of film-coated tablets under stress conditions is significantly lower in Example 1 than in Reference Example 2 (without wishing to be bound by theory, assumed to be due to the non-hygroscopicity (or low hygroscopicity) properties provided by mannitol in the compositions of the present invention), which ensures adequate quality (mechanical stability) of pharmaceutical compositions of the invention within the shelf life. With a higher content (higher weight percentage) of magnesium stearate present in examples 3 to 9 (said examples have a higher percentage of magnesium stearate than reference examples), we also eliminated the adhesion of the tableting composition to the punches, which means a significantly improved processability in long-term tableting, while maintaining adequate dissolution and thus bioavailability.

### Example 10 - Crystallization of apremilast

### Example 10a - Comparative crystallization examples

As may be seen when comparing the crystallization examples according to the invention (Example 10b) with comparative Examples (Example 10a), apremilast prepared according to the crystallization examples according to the invention has a considerable more advantageous morphology.

### Crystallization of apremilast at room temperature with rapid addition of ethanol. Comparative crystallization example 1

Apremilast (6.07 g) was dissolved in acetone (30 mL) at reflux. Ethanol (60 mL) was added to the solution. After the addition of ethanol, the temperature of the mixture was maintained at 23 °C and stirred for 17 hours. The suspension was filtered on a vacuum filter and the product was washed with ethanol (15 mL). The precipitate was dried in a vacuum dryer at 40 °C for 16 hours. 5.73 g of apremilast is obtained in the form of spherical agglomerates composed of small needle like primary particles.

| | **Comparative crystallization example 1** | |
|---|---|---|
| | width | height |
| Mean [µm] | 1.7 | 0.6 |

### Crystallization of apremilast at room temperature with gradual addition of ethanol. Comparative crystallization example 2

Apremilast (6.96 g) was dissolved in acetone (30 mL) at reflux. Ethanol (6 mL) was added to the solution at 25 °C and the solution was seeded with apremilast crystals (25 mg) followed by addition of ethanol (56 mL) at 25 °C over three hours. Formed suspension was stirred at 25 °C for 17 hours. The suspension was filtered on a vacuum filter and the product was washed with ethanol (15 mL). The precipitate was dried in a vacuum dryer at 40 °C for 16 hours and 6.27 g of apremilast was obtained in the form of spherical agglomerates composed of small needle like primary particles.

| | **Comparative crystallization example 2** | |
|---|---|---|
| | width | height |
| Mean [µm] | 1.6 | 0.7 |

### Crystallization of apremilast at elevated temperature and immediate cooling after addition of ethanol. Comparative crystallization example 3

Apremilast (25 g) was dissolved in acetone (107.5 mL) at reflux temperature. The solution was cooled to 40 °C and seeded with apremilast crystals (250 mg). Ethanol (215 mL) at ambient temperature was added over 20 minutes. During addition of ethanol temperature of suspension dropped to about 27 °C. Suspension was cooled to 0 °C within 5 h. Product was isolated by vacuum filtration and the cake was washed with 25 mL of ethanol. The precipitate was dried in a vacuum dryer at 40 °C for 16 hours and 23.45 g of apremilast was obtained in the form of spherical agglomerates composed of small needle like primary particles.

| | **Comparative crystallization example 3** | |
|---|---|---|
| | width | height |
| Mean [µm] | 2.4 | 0.7 |

### Example 10b - Crystallization examples according to the method of the present invention

### Crystallization example 1

### Recrystallization in acetone:ethanol (1:2), prolonged stirring for 21 h at 40 °C, cooling to 0 °C (6 h), 180 rpm

Apremilast (400 g) was dissolved in acetone (1720 mL) at reflux temperature in reactor with overhead stirrer. Solution was cooled to 40 °C and seeded with apremilast crystals (4 g). After seeding preheated ethanol (3440 mL) at 40 °C was added into acetone solution in 30 minutes and stirred for 21 h at 40 °C. The suspension was slowly cooled to 0 °C and apremilast was isolated by vacuum filtration. The product was dried in a vacuum dryer at 40 °C for 16 hours and 365.9 g of apremilast was obtained that was substantially free of small needle like primary particles.

| | **Crystallization example 1** | |
|---|---|---|
| | width | height |
| Mean [µm] | 7.6 | 3.3 |

### Crystallization example 2

### Recrystallization in acetone:ethanol (1:2), longer stirring time at 44 °C, cooling to 0 °C

Apremilast (6 g) was dissolved in acetone (25.8 mL) at reflux temperature in reactor with overhead stirrer. Solution was cooled to 44 °C and seeded with apremilast crystals (60 mg). After seeding preheated ethanol (51.6 mL) was added over 5 minutes and suspension was stirred for 20 h at 44 °C. The suspension was slowly cooled to 0 °C and apremilast was isolated by vacuum filtration. The product was dried in a vacuum dryer at 40 °C for 16 hours and 5.2 g of apremilast was obtained that was substantially free of small needle like primary particles.

| | **Crystallization example 2** | |
|---|---|---|
| | width | height |
| Mean [µm] | 11.5 | 7.0 |

### Crystallization example 3

### Recrystallization to acetone / ethanol (1: 2), heating to reflux, longer cooling time to Tj = 40 °C, long cooling to 0 °C (20h)

Apremilast (25 g) was dissolved in acetone (107.5 mL) at reflux temperature in reactor with overhead stirrer. Solution was cooled to 44 °C and inoculated with apremilast crystals (250 mg). After seeding preheated ethanol (215 mL) was added over 30 minutes at 44 °C. At the end of the addition a cloudy suspension was observed that was heated to 65 °C. Still partially cloudy suspension was cooled to 57 °C and stirred for 2h followed by cooling of solution to 40 °C in 10 hours. The suspension was slowly cooled to 0 °C and apremilast was isolated by vacuum filtration. The product was dried in a vacuum dryer at 40 °C for 16 hours and 23.56 g of apremilast was obtained that was substantially free of small needle like primary particles.

| | **Crystallization example 3** | |
|---|---|---|
| | width | height |
| Mean [µm] | 6.7 | 2.5 |

### Crystallization example 4

### Recrystallization to acetone / ethanol (1: 2), longer stirring time (20h) at 50 °C, cooling to 0 °C (6h)

Apremilast (25 g) was dissolved in acetone (107.5 mL) at reflux temperature in reactor with overhead stirrer. Solution was cooled to 50 °C and seeded with apremilast crystals (250 mg). After seeding preheated ethanol (215 mL) was added over 30 minutes at 50 °C and suspension was stirred for 20 h at 50 °C. The suspension was slowly cooled to 0 °C and apremilast was isolated by vacuum filtration. The product was dried in a vacuum dryer at 40 °C for 16 hours and 20.75 g of apremilast was obtained that was substantially free of small needle like primary particles.

| | **Crystallization example 4** | |
|---|---|---|
| | width | height |
| Mean [µm] | 20 | 7.7 |

### Further Crystallization examples

Crystallization experiments repeating Crystallization experiments 1 to 4 of the invention supra were carried out, except of adding apremilast seeding crystals.

As may be seen from the examples above, it is particularily advantageous to apply the following parameters during the solvent-antisolvent crystallization:
- Addition temperature of ethanol in the range from 35 to 55 °C, preferably from 40 to 50 °C,
- Stirring time at crystallization temperature range, especially first crystallization temperature range, for from 5 to 25 hour preferably from 10 to 20 hour

Both, advantageous addition and stirring temperature, as well as an advantagously long stirring time are advantageous for obtaining apremilast that is substantially free of thin needle like particles as separate fraction, as adhered fraction on bigger particles or in the form of no resistant spherical agglomerates.

As may be seen from the SEM images shown in Figures 1 and 2, Apremilast obtained according to the crystallization method of the present invention is substantially free of needle like thin particles. For apremilast obtained according to the crystallization method of the present invention, the mean dimension of particles width is advantageously not less than 5.0 µm, preferably not less than 6 µm, more preferably not less than 8 µm, most preferably not less than 10 µm, e.g. in the range of from 5.0 µm to 10 µm, and the mean dimension of particles height is advantageously not less than 2.0 µm, preferably not less than 3 µm, more preferably not less than 5 µm, most preferably not less than 7 µm, e.g. in the range of from 2.0 µm to 7 µm. In an embodiment, the mean dimension of particle width and the mean dimension of particle height can be average particle width and average particle height, which can be e.g. determined based on particles number average (which can be e.g. determined from SEM images using a computer program, as described in the section entitled "Analytical methods" below).

The particle size can be determined from SEM images. Methods for determining particle size from SEM images are known in the art. The method used by the inventors is described herein in the section entitled "Analytical methods".

### Example 11 - Experiments concerning pharmaceutical compositions

Pharmaceutical compositions of the present invention provide several advantageous properties:
- Pharmaceutical compositions of the present invention comprising apremilast and mannitol show advantageous physical properties, even when using apremilast crystallized according to prior art (Table 4).
- Pharmaceutical compositions of the present invention comprising apremilast prepared by a preparation process according to the present invention, and mannitol show even more advantageous physical properties, especially even better flow properties, and an advantageous maintaining of compressibility and AV values (Table 4).
- Pharmaceutical compositions of the present invention show an advantageous stability of the dissolution profile and an advantageous stability of the dissolution properties even after storage and after exposure to stress conditions; pharmaceutical compositions of the present invention show in this respect properties even superior to the properties of the commercially available composition Otezla^{®}.
- Pharmaceutical compositions of the present invention comprising mannitol and using apremilast according to prior art show an improved dissolution stability after 1 month of exposure to stress conditions compared to the commercially available composition Otezla^{®}. An even superior dissolution stability is observed, when using apremilast crystallized according to prior art.

### a) Storage stability and dissoluton profile of tablets comprising apremilast prepared by a preparation process according to the present invention (using apremilast prepared with a crystallization method not according to the invention)

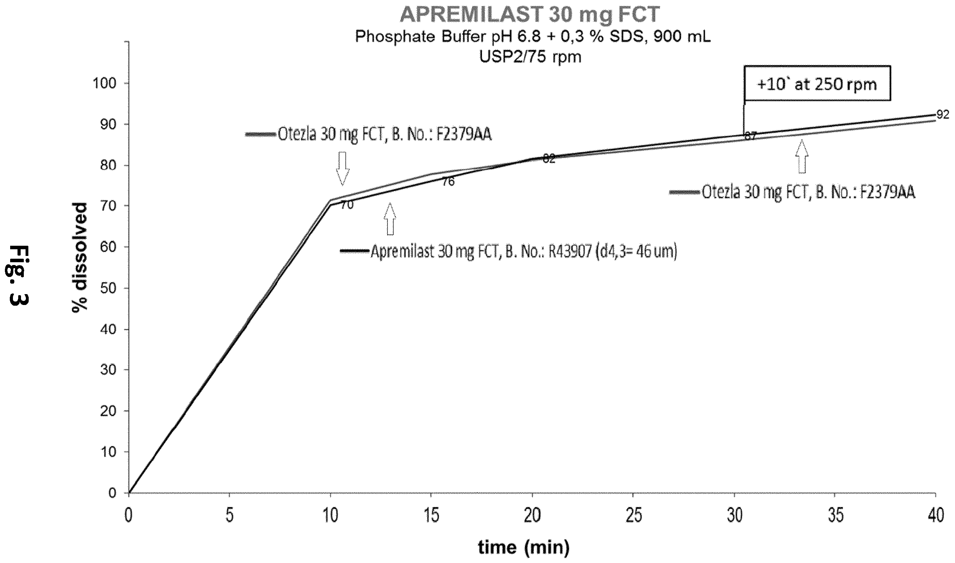

By a non-optimized method of crystallization (i.e. not according to the crystallization method of the invention) of the active ingredient, apremilast particles are obtained, which show a dissolution profile in the formulation (Example 3), which is comparable to commercially available Otezla^{®} tablets (Figure 3). The dissolution profile was determined under the following experimental conditions: phosphate buffer pH 6.8 + 0.3 % SDS, 900 mL, using an USP2 apparatus, 75 rpm.

This is highly surprising, as the composition of Example 3 is a composition free of lactose. This is further highly surprising as a crystallization method not according to the invention was used which contains a significant amount of needle-like apremilast particles.

### b) Presence of mannitol

The presence of mannitol has an advantageous effect on storage stability of tablets and compacts comprising apremilast and mannitol.

In experiments, compacts comprising mannitol and apremilast in a 1:1 ratio were prepared. After exposure for 3 days to 80% relative humidity, when using mannitol Parteck^{®} M100, the advantage observed is that merely a small increase in diameter occurs. Compacts comprising Lactose monohydrate show a disadvantageous, considerably larger increase in diameter (relative change in particle diameter: 20 %). Without wishing to be bound to theory, it is assumed that said smaller increase contributes to the advantageous property of the mannitol-containing compositions of the present invention which prevent or significantly reduce hardening of the apremilast particles, especially when compared to pharmaceutical compositions comprising lactose (in particular lactose monohydrate).

**Table 5: Change in average particle diameter of compacts of Inventive Example 9 and both mannitols in a 1:1 ratio after exposure for 3 days to 80% relative humidity.**

| **Excipient** | **Relative change in average particle diameter in %** |
|---|---|
| Mannitol Parteck^{®} M100 | + 15% |
| Lactose monohydrate (FlowLac^{®} 100) | + 20% |

### c) Comparative experiments comparing tablets according to the present invention with commercially available tablets

Tablets according to present invention were compared with commercially available Otezla^{®} tablets. In a first set of experiments, two tablet embodiments (tablet composition prepared according to Example 5 in combination with apremilast prepared by a method not according to the invention), Otezla^{®} (F2285AA) were subjected to a dissolution experiment and the extent of dissolution after 30 minutes was determined.

In a second set of experiments, said two tablet embodiments were subjected subjected to a storage at elevated temperature (50 °C) and high relative humidity (75%) during 1 month, before the same dissolution experiment was carried out. Subsequently, the slowdown of the dissolution (in %) was determined on the basis of the two sets of comparative experiments carried out.

Table 6 shows that tablets according to Example 5 merely show a very small slowdown of dissolution, when compared to Otezla^{®} tablets. This is highly surprising, as the tablets of Example 5 merely comprise apremilast, which was not prepared using the process of the present invention, and are free of the excipient lactose. It is therefore expected that tablets of the present invention will show an improved shelf life, and/or an improved (higher) temperature limit for storage conditions. Table 6 shows that even if apremilast is prepared according to prior art, the formulation with mannitol is still better than formulation with lactose (Otezla^{®}) (without wishing to be bound by theory, this is expected to be due to protection imparted by mannitol against hardening of apremilast particles).

**Table 6: Slowdown of the dissolution - Comparison of Example 5 versus Otezla^{®}**

| **Example** | **Slowdown of the dissolution in % after 30 min (dissolution period) according to the initial analysis at elevated temperature (50 °C) and relative humidity (75%) after** |
|---|---|
| | **1 month** |
| Example 5 | -3% |
| Otezla^{®} (F2285AA) | - 5 % |

### d) Storage stability and dissoluton profile of tablets comprising apremilast prepared by a preparation process according to the present invention

Furthermore, tablets according to present invention comprising apremilast crystallized according to a crystallization process of the present invention (tablet composition prepared according to Inventive Example 6 in combination with apremilast prepared by a method according to the invention) are subjected to stress conditions (elevated temperature (50 °C) and relative humidity (75%), 14 days (14 days and 1 month are considered to be equivalent with regards to dissolution drop). After 14 days under stress conditions a dissolution experiment is carried out, which compares tablets subjected to stress conditions with tablets not subjected to stress conditions. Merely a small slowdown of the dissolution is observed for tablets according to present invention comprising apremilast crystallized according to a crystallization process of the present invention in spite of storage under stress conditions. It is therefore expected that tablets of the present invention comprising apremilast obtained by a crystallization according to the present invention will show an advantageous shelf life, and/or an advantageous (high) temperature limit for storage.

If an active substance crystallized according to an optimized process (a method for crystallizing apremilast according to the present invention) is used in the formulation and mannitol (Example 6) is used in the composition, the dissolution drop is unexpectedly reduced even further (-1 %) (Table 7).

**Table 7: Slowdown of the dissolution for Example 6 in 30 min according to the initial analysis at elevated temperature (50 °C) and relative humidity (75%) after 14 days.**

| **Example** | **Slowdown of the dissolution in % after 30 min according to the initial analysis at 50 °C/75 % RH in 14 days** |
|---|---|
| Example 6 using apremilast crystallized according to a crystallization process of the present invention | -1% |

The optimized process of crystallization of the active ingredient apremilast according to the present invention leads to more mechanically resistant and larger primary particles with a minimal proportion of the powder fraction of needle like primary particles. Thus, the active ingredient crystallized in the range of optimized conditions is more resistant to hardening, as confirmed by experiments under stress conditions (high temperature and high relative humidity).

### Analytical methods

The BET surface area determination for mannitol can be carried out using instruments from Micromeritics Instrument Corporation (USA) [e.g. ASAP 2420 or equivalent]. The BET surface area determination can be carried out by a volumetric method using nitrogen (sample weight e.g. 3 g, preferably subjecting the sample to a sample preparation (e.g. heating at 3.0°C/min. to the target temperature of 50°C, 10 hours at 50°C).

The pore volume determination of mannitol can be carried out using instruments from Micromeritics Instrument Corporation (USA) [e.g. ASAP 2420 or equivalent]. The pore volume can be determined from nitrogen adsorption and desorption isotherms with application of Barrett-Joyner-Halenda (BJH) method (sample weight e.g. 3 g, preferably subjecting the sample to a sample preparation (e.g. heating at 3.0°C/min. to the target temperature of 50°C, 10 hours at 50°C).

The apremilast particle size is determined from SEM images. For determining apremilast particle size, apremilast particles were characterized using SEM images. Samples were analyzed by scanning electron microscopy (SEM). Images of the samples taken with SEM are entered into the program (analySIS PRO (Olympus)), which allows us to measure individual particles. A certain number of particles is measured and from all the measurements made, program calculates average values, as well as the minimum and the maximum.

Particles in the samples obtained by the present invention are columnar in shape (long, thin particle with a width and thickness that are greater than those of an acicular particle (needle-like particle)). From all 3 dimensions (length, width and height), we measured the two dimensions where the differences between the particles of apremilast obtained by the crystalization of the present invention and the particles from comperative examples were the most pronounced :
- width: the shorter side of the flat surface (b)
- height: the part of the rectangular prism that rises up (c); reference is made to Table 8 below:

**Table 8:**

| | |
|---|---|
| 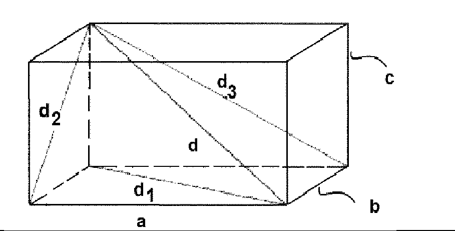 | width: the shorter side of the flat surface (b), height: the part of the rectangular prism that rises up (c) |

Dissolution experiments can be performed in 900 mL of a dissolution medium containing 0.05 mol/L sodium phosphate at pH 6.8 and 0.3 wt.-% SDS (sodium dodecylsulfate) (based on the weight of the dissolution medium) at 37°C using USP apparatus 2 (paddles) at a rotation speed of 75 rpm. The samples taken during dissolution test can be analyzed e.g. by HPLC (high performance liquid chromatography). The amount of dissolved apremilast is determined, e.g. using HPLC, by using external standard solution with known concentration. The area under the response of the standard solution is then compared to area under the response of apremilast in the sample, and the amount of apremilast of the sample is then calculated, considering the concentration of standard solution and its response.

## Claims

1. Pharmaceutical composition, said pharmaceutical composition being or comprising a mixture, said mixture comprising:
3 - 20 wt.%, preferably 5 - 15 wt.%, more preferably 7.5 - 12.5 wt.% of apremilast, based on the total weight of said mixture,
40 - 90 wt.%, preferably 50 - 80 wt.%, more preferably 55 - 75 wt.% of mannitol, based on the total weight of said mixture,
0.5 - 3 wt.%, preferably 1 - 2 wt.%, more preferably 1.2 - 1.8 wt.% of magnesium stearate, based on the total weight of said mixture, and
0.5 - 6 wt.%, preferably 1 - 5 wt.%, more preferably 2 - 4 wt.% of a disintegrant, based on the total weight of said mixture.

2. Pharmaceutical composition according to claim 1, wherein said mixture is a compressed mixture, in particular tablet.

3. Pharmaceutical composition according to any one of the preceding claims, wherein said mixture further comprises 0.1 - 45 wt.%, preferably 10 - 35 wt.%, more preferably 15 - 30 wt.% of a filler, based on the total weight of the mixture, said filler being a filler other than a filler comprising or consisting of mannitol.

4. Pharmaceutical composition according to claim 3, wherein said filler is or comprises microcrystalline cellulose.

5. Pharmaceutical composition according to any one of the preceding claims, wherein said mannitol is or comprises spray-dried mannitol.

6. Pharmaceutical composition according to any one of the preceding claims, wherein said mannitol is or comprises mannitol having a surface area in the range of 1.0 - 5.0 m²/g, preferably 2.5 - 4.0 m²/g, more preferably 2.6 - 3.9 m²/g, even more preferably in the range of 2.8 - 3.0 m²/g.

7. Pharmaceutical composition according to any one of the preceding claims, wherein said mannitol is or comprises mannitol having a pore volume in the range of 0.01 - 0.1 cm³/g, preferably in the range of 0.01 - 0.07 cm³/g, more preferably in the range 0.01 - 0.02 cm³/g or in the range 0.02 - 0.07 cm³/g.

8. Pharmaceutical composition according to any one of the preceding claims, which pharmaceutical composition is free of lactose.

9. Pharmaceutical composition according to any one of the preceding claims, wherein said mixture comprising apremilast, mannitol, magnesium stearate and disintegrant has a weight ratio between apremilast and magnesium stearate (apremilast : magnesium stearate), which weight ratio is 100:30 or less, and/or
wherein said mixture comprising apremilast, mannitol, magnesium stearate and disintegrant has a weight ratio between mannitol and magnesium stearate (mannitol: magnesium stearate), which weight ratio is 600:30 or less, and/or
wherein said mixture comprising apremilast, mannitol, magnesium stearate and disintegrant has a weight ratio between apremilast and magnesium stearate (apremilast : mannitol), which weight ratio is 1:9 or less.

10. Process for preparing a pharmaceutical composition according to any one of the preceding claims, said process comprising:
a) preparing a mixture comprising:
3-20 wt.%, preferably 5 - 15 wt.%, more preferably 7.5 - 12.5 wt.% of apremilast, based on the total weight of said mixture,
40-90 wt.%, preferably 50 - 80 wt.%, more preferably 55 - 75 wt.% of mannitol, based on the total weight of said mixture,
0.5 - 3 wt.%, preferably 1 - 2 wt.%, more preferably 1.2 - 1.8 wt.% of magnesium stearate, based on the total weight of said mixture, and
0.5 - 6 wt.%, preferably 1 - 5 wt.%, more preferably 2 - 4 wt.% of a disintegrant, based on the total weight of said mixture,
b) compressing said mixture to obtain a compressed mixture, in particular tablet,
c) optionally coating the compressed mixture, in particular tablet, with a coating, in particular a film coating.

11. Method for crystallizing apremilast, said method comprising the following steps:
a) preparing a solution comprising apremilast and a solvent, said solvent being or comprising acetone,
b) optionally adding seeding material to the solution comprising apremilast and a solvent to obtain a seeding material containing mixture,
c) adding an antisolvent and optionally adding seeding material to the solution comprising apremilast prepared in step a) or to the seeding crystal containing mixture optionally prepared in step b) during an antisolvent addition time period of from 1 minute to 20 hours, preferably of from 10 minutes to 1 hour, to obtain a mixture comprising apremilast, solvent, and antisolvent,
said antisolvent being or comprising ethanol,
d) optionally increasing the temperature of the mixture comprising apremilast, solvent, and antisolvent obtained in step c) to a temperature in the temperature range up to 65°C,
e) subjecting the mixture comprising apremilast, solvent, and antisolvent to agitation at a temperature in a first crystallization temperature range of from 35 °C to 55 °C, preferably from 40°C to 50°C, during a first crystallization period of from 5 hours to 25 hours, preferably of from 10 hours to 20 hours, to obtain a suspension comprising apremilast,
f) decreasing the temperature of the suspension comprising apremilast to a temperature in a second crystallization temperature range of from -20°C to 20°C, preferably of from -10°C to 10°C,
g) separating crystalline apremilast from the suspension comprising apremilast,
h) optionally drying the separated crystalline apremilast.

12. Method for crystallizing apremilast according to claim 11, wherein the mixture comprising apremilast, solvent, and antisolvent, especially the mixture comprising apremilast, solvent and antisolvent subjected to agitation in step e), comprises ethanol and acetone in a ratio ethanol to acetone (ethanol : acetone) which can be in the range of from 1:5 to 5: 1, preferably of from 1: 3 to 3: 1, more preferably in an amount of 1:2 to 2:1, especially in the range of from 2:1 to 5:1, further especially in the range of from 2:1 to 3:1, and/or
wherein in step c) said solution comprising apremilast prepared in step a) or said seeding crystal containing mixture optionally prepared in step b) has a temperature in the temperature range of from 35°C to 55°C, preferably from 40°C to 50°C, and/or wherein in step c) during the antisolvent addition time period, the temperature of the mixture comprising apremilast, solvent, and antisolvent has a temperature in the temperature range of from 35°C to 55°C, preferably from 40°C to 50°C, and/or
wherein the antisolvent added in step c) has a temperature in the range of from 35 °C to 55 °C, preferably from 40°C to 50°C.

13. Apremilast obtainable by the method for crystallizing apremilast according to claim 11 or 12.

14. Apremilast according to claim 13 or the composition according to any one of claims 1 to 10 for use as a medicament.

15. Apremilast according to claim 13 or the composition according to any one of claims 1 to 10 for use in a method of treating or preventing a disease or disorder selected from psoriasis, Behçet's disease, arthritis other than rheumatoid arthritis and combinations thereof.
